# EUROPÄISCHE PATENTSCHRIFT

(11) **EP 0 594 134 B1**
(45) Veröffentlichungstag und Bekanntmachung des Hinweises auf die Patenterteilung: **07.01.1999**
(21) Anmeldenummer: 93116890.0
(22) Anmeldetag: 19.10.1993
(51) Int. Cl.: G01N 33/72, G01N 21/01, C03C 4/02

(54) **Kalibriermittel zur photometrischen Bilirubinbestimmung**
Calibrator for photometrical bilirubin assay
Calibrateur pour l'essai photométrique de bilirubin

(30) Priorität: 20.10.1992 DE 4235311
(43) Veröffentlichungstag der Anmeldung: 27.04.1994
(73) Patentinhaber: Pfaff, Dieter, 86633 Neuburg (DE)
(72) Erfinder: Pfaff, Dieter, 86633 Neuburg (DE)
(74) Vertreter: Kirschner, Klaus Dieter, Dipl.-Phys.

(56) Entgegenhaltungen:
- US-A- 3 942 899
- DATABASE WPI Week 9247, Derwent Publications Ltd., London, GB; AN 92-388488 & SU-A-1 671 625 (GLASS RES INST ET AL.) 23. August 1991
- DATABASE WPI Week 8946, Derwent Publications Ltd., London, GB; AN 89-335802 & JP-A-1 249 633 (TOSHIBA GLASS KK) 4. Oktober 1989
- DATABASE WPI Week 9136, Derwent Publications Ltd., London, GB; AN 91264863 & SU-A-1 596 255 (BELO MATERNITY CHIL) 30. September 1990

## Beschreibung

Die Erfindung betrifft Mittel zur Kalibrierung bei der photometrischen Bilirubinbestimmung sowie einen Photometer.

Bilirubin tritt als Abbauprodukt des Blutfarbstoffs in der Galle und insbesondere bei Hepatitis im Blut in erhöhtem Maße auf. In diesem Zusammenhang wird insbesondere der Bilirubingehalt im Blutserum Neugeborener überprüft, um das Ausmaß einer eventuell auftretenden Neugeborenen-Hepatitis abschätzen zu können. Hierbei können bei Neugeborenen Bilirubinwerte auftreten, die wesentlich größer sind als die bei Erwachsenen.

Die Bestimmung des Bilirubinwertes erfolgt in der Praxis im wesentlichen photometrisch. Photometrische Analyse-Methoden erfordern zur Kalibrierung geeignete Kalibriermittel (Standard). Die eigentliche Messung beruht auf der Bestimmung der Absorption einer bestimmten Lichtwellenlänge bei Durchstrahlung der zu analysierenden Probe. Die gemessene Absorption wird zur Absorption, die bei der Messung des Standards bestimmt wurde, in Beziehung gesetzt und so der quantitative Gehalt der zu bestimmenden Substanz in der Lösung bestimmt.

Als Standard zur Kalibrierung bei der photometrischen Bilirubinbestimmung dienen bislang Blutseren, welche aus dem Blut von Erwachsenen gewonnen wurden. So ist beispielsweise aus DATABASE WPI, Week 9136, Derwent Publc. Ltd., London, GB, AN 91264863 & SU-A-1 596 255 bekannt, Bilirubin in kapillarem Blut photometrisch mit einer Blutprobe zu bestimmen, die verdünnt ist, wobei das relative Volumen des Plasmas mit einer Formel bestimmt wird. Diese Kontroll- oder Standardseren zur Bilirubinbestimmung haben jedoch den Nachteil, daß sie in ihrer Zusammensetzung und Standardisierung von Charge zu Charge und von Hersteller zu Hersteller variieren.

Dies führt häufig zu Verwirrungen bei der Interpretation von Meßergebnissen oder zu Fehlinterpretationen.

Ein weiteres Problem der Kalibrierung bei der photometrischen Bilirubinbestimmung von Neugeborenenseren resultiert aus der Tatsache, daß Kalibriermittel auf der Basis von Erwachsenenseren oft ungeeignet sind, da letztere wesentlich geringere Bilirubinwerte aufweisen und somit die Linearität der Kalibrierung bei hohen Werten nicht mehr gewährleistet ist.

Eine weitere Fehlerquelle ergibt sich daraus, daß die optischen Eigenschaften der Kontrollseren unter dem Einfluß von Licht sowie durch Einfrieren und Auftauen beeinflußt werden. Um die Haltbarkeit derartiger Seren zu verlängern, ist es jedoch notwendig, diese gekühlt oder gefroren zu lagern. Insgesamt erfordert die begrenzte Haltbarkeit der Kontrollseren eine ständige Vorratshaltung sowie die Notwendigkeit, diese immer wieder nachzukaufen.

Für die praktische Durchführung der photometrischen Bilirubinbestimmung wäre es daher eine enorme Erleichterung, wenn ein Kalibriermittel zur Verfügung stehen würde, welches lange haltbar ist und eine Kalibrierung mit hoher Zuverlässigkeit erlaubt.

Aufgabe der vorliegenden Erfindung ist es daher, ein kostengünstiges und haltbares Kalibriermittel zur photometrischen Bilirubinbestimmung sowohl bei Erwachsenenen als auch bei Neugeborenen anzugeben und die entsprechenden photometrischen Meßgeräte zu verbessern.

Diese Aufgabe wird erfindungsgemäß durch die in dem kennzeichnenden Teil des Patentanspruchs 1 bzw. des Patentanspruches 5 enthaltenen Merkmale gelöst, wobei zweckmäßige Weiterbildungen der Erfindung durch die in den Unteransprüchen angegebenen Merkmale gekennzeichnet sind.

In einer vorteilhaften Ausgestaltung handelt es sich hierbei um ein Gelbglas, welches folgende bevorzugte Zusammensetzung aufweist:
- 66,0 - 68,0 Gew.% SiO₂, vorzugsweise 66,91 Gew.% SiO₂,
- 10,0 - 11,0 Gew.% Na₂O, vorzugsweise 10,88 Gew.% Na₂O,
- 6,0 - 7,0 Gew.% CaO, vorzugsweise 6,79 Gew.% CaO,
- 3,0 - 4,0 Gew.% Al₂O₃, vorzugsweise 3,46 Gew.% Al₂O₃,
- 3,0 - 4,0 Gew.% B₂O₃, vorzugsweise 3,18 Gew.% B₂O₃,
- 2,0 - 2,5 Gew.% ZnO, vorzugsweise 2,07 Gew.% ZnO,
- höchstens 0,1 Gew.% Fe₂O₃, vorzugsweise 0,080 Gew.% Fe₂O₃,
- höchstens 0,1 Gew.% TiO₂, vorzugsweise 0,083 Gew.% TiO₂,
- höchstens 0,06 Gew.% MgO, vorzugsweise 0,050 Gew.% MgO,
- höchstens 0,03 Gew.% BaO, vorzugsweise 0,025 Gew.% BaO,
- höchstens 0,02 Gew.% PbO, vorzugsweise 0,017 Gew.% PbO,
- höchstens 0,02 Gew.% Sb₂O₃,
- höchstens 0,01 Gew.% SrO, und
- höchstens 0,006 Gew.% Li₂O.

Dieses Glas ist für die Bilirubinbestimmung bei Neugeborenen geeignet.

In einer weiteren vorteilhaften Ausgestaltung weist das Gelbglas folgende bevorzugte Zusammensetzung auf:
- 66,0 - 68,0 Gew.% SiO₂, vorzugsweise 66,91 Gew.% SiO₂,
- 10,0 - 11,0 Gew.% Na₂O, vorzugsweise 10,88 Gew.% Na₂O,
- 6,0 - 7,0 Gew.% CaO, vorzugsweise 6,79 Gew.% CaO,
- 3,0 - 4,0 Gew.% Al₂O₃, vorzugsweise 3,46 Gew.% Al₂O₃,
- 3,0 - 4,0 Gew.% B₂O₃, vorzugsweise 3,18 Gew.% B₂O₃,
- 2,0 - 2,5 Gew.% ZnO, vorzugsweise 2,07 Gew.% ZnO,
- höchstens 0,1 Gew.% Fe₂O₃, vorzugsweise 0,01 Gew.% Fe₂O₃,
- höchstens 0,1 Gew.% TiO₂, vorzugsweise 0,01 Gew.% TiO₂,
- höchstens 0,06 Gew.% MgO, vorzugsweise 0,050 Gew.% MgO,
- höchstens 0,03 Gew.% BaO, vorzugsweise 0,025 Gew.% BaO,
- höchstens 0,02 Gew.% PbO, vorzugsweise 0,005 Gew.% PbO,
- höchstens 0,02 Gew.% Sb₂O₃,
- höchstens 0,01 Gew.% SrO, und
- höchstens 0,006 Gew.% Li₂O.

Dieses Glas ist für die Bilirubinbestimmung bei Erwachsenen geeignet.

Die beiden Glaszusammensetzungen unterscheiden sich in dem Gehalt an TiO₂, PbO und Fe₂O₃, die in folgenden Bereichen liegen:

| | |
|---|---|
| TiO₂ | 0,01 - 0,083 Gew. % |
| PbO | 0,005 - 0,017 Gew. % |
| Fe₂O₃ | 0.01 - 0,080 Gew. %, |

wobei eine erfindungsgemäße Kapillare bei Bedarf auch eine Zusammensetzung haben kann, bei der die Werte für die vorstehend genannten Bestandteile zwischen den beanspruchten Werten liegen kann, wenn eine Patientengruppe untersucht werden soll, deren zu erwartende Bilirubinwerte zwischen denen von Erwachsenen und denen von Neugeborenen liegen.

In einer weiteren bevorzugten Ausgestaltungsform sind die Kalibriermittel, insbesondere in ihren geometrischen Abmessungen derart gestaltet, daß sie problemlos bei den marktüblichen Photometern zur Bilirubinbestimmung eingesetzt werden können. Sie sind insbesondere in Form von Kapillaren mit einem Kapillardurchmesser von 1,5 - 2,0 mm gebildet.

Ferner können Photometer in vorteilhafter Weise mit den erfindungsgemäßen Kalibriermitteln versehen sein, so daß deren Kalibrierung wesentlich erleichtert ist. In diesem Zusammenhang können die relevanten Kalibrier- und/oder Differenzwerte über eine Datenverarbeitungskomponente des Geräts verarbeitet, gespeichert und abgerufen werden.

Unter Verwendung der erfindungsgemäßen Kalibriermittel stellt sich die Kalibrierung des Photometers nunmehr wie folgt dar. Zunächst erfolgt eine Messung und Einstellung des Photometers mit einem herkömmlichen Bilirubinstandard. Beim zweiten Schritt wird der Wert der erfindungsgemäßen Eich-Kapillare festgestellt. Daraus ergibt sich ein Differenzwert für die Eich-Kapillare, welcher gerätespezifisch und damit fortan gültig ist.

Insgesamt ist festzustellen, daß sich die erfindungsgemäßen Kalibriermittel insbesondere durch ihre Farbstabilität, d.h. ihre Unabhängigkeit vom umgebenden Licht und ihre Langzeitstabilität sowie durch ihre einfache Handhabung auszeichnen. Umfangreiche Meßreihen haben ergeben, daß unter Verwendung dieser Kalibriermittel die Ergebnisse von Bilirubinmetern in einem weiten Konzentrationsbereich erheblich verbessert werden konnten.

Die folgenden Darstellungen belegen den Fortschritt, welcher unter Verwendung der erfindungsgemäßen Kalibriermittel erreicht wurde. Hierin zeigen:
- Fig.1:: die Linearität eines Bilirubinmeters, welches in herkömmlicher Weise kalibriert wurde, und
- Fig.2:: die Linearität eines Bilirubinmeters, welches unter Verwendung der erfindungsgemäßen Kalibriermittel kalibriert wurde.

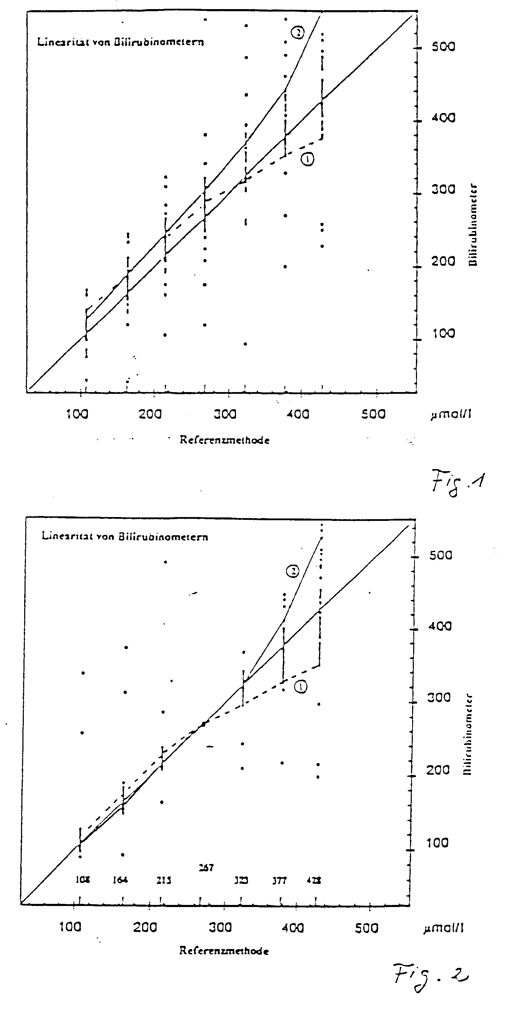

In den Figuren 1 und 2 ist auf der Abszisse jeweils der vorgegebene Konzentrationswert der Probe und auf der Ordinate der photometrisch gemessene Wert der Probe dargestellt. Sowohl bei herkömmlicher Kalibrierung (Fig.1) als auch bei der Verwendung der erfindungsgemäßen Kalibriermittel ergibt sich, daß oberhalb einer bestimmten Konzentration, welche bei 300 µmol/l liegt, die Zuverlässigkeit der Meßmethode nicht mehr gewährleistet ist. Unterhalb dieses Wertes zeigt sich jedoch bei Vergleich der Figuren 1 und 2, daß bei Verwendung der erfindungsgemäßen Kalibriermittel eine wesentlich verbesserte Linearität der Meßmethode gewährleistet ist. Dies führt insgesamt zu wesentlich genaueren Meßwerten.

## Patentansprüche

1. Kalibriermittel zur photometrischen Bilirubinbestimmung, **dadurch gekennzeichnet, daß** die Kalibriermittel aus Glas gebildet sind.

2. Kalibriermittel nach Anspruch 1, **dadurch gekennzeichnet, daß** die Kalibriermittel aus einem Glas gebildet sind, das folgende Zusammensetzung aufweist:
- 66,0 - 68,0 Gew.% SiO₂, vorzugsweise 66,91 Gew.% SiO₂,
- 10,0 - 11,0 Gew.% Na₂O, vorzugsweise 10,88 Gew.% Na₂O,
- 6,0 - 7,0 Gew.% CaO, vorzugsweise 6,79 Gew.% CaO,
- 3,0 - 4,0 Gew.% Al₂O₃, vorzugsweise 3,46 Gew.% Al₂O₃,
- 3,0 - 4,0 Gew.% B₂O₃, vorzugsweise 3,18 Gew.% B₂O₃,
- 2,0 - 2,5 Gew.% ZnO, vorzugsweise 2,07 Gew.% ZnO,
- höchstens 0,1 Gew.% Fe₂O₃, vorzugsweise 0,080 Gew.% Fe₂O₃,
- höchstens 0,1 Gew.% TiO₂, vorzugsweise 0,083 Gew.% TiO₂,
- höchstens 0,06 Gew.% MgO, vorzugsweise 0,050 Gew.% MgO,
- höchstens 0,03 Gew.% BaO, vorzugsweise 0,025 Gew.% BaO,
- höchstens 0,02 Gew.% PbO, vorzugsweise 0,017 Gew.% PbO,
- höchstens 0,02 Gew.% Sb₂O₃,
- höchstens 0,01 Gew.% SrO, und
- höchstens 0,006 Gew.% Li₂O.

3. Kalibriermittel nach Anspruch 1, **dadurch gekennzeichnet, daß** die Kalibriermittel aus einem Glas gebildet sind, das folgende Zusammensetzung aufweist:
- 66,0 - 68,0 Gew.% SiO₂, vorzugsweise 66,91 Gew.% SiO₂,
- 10,0 - 11,0 Gew.% Na₂O, vorzugsweise 10,88 Gew.% Na₂O,
- 6,0 - 7,0 Gew.% CaO, vorzugsweise 6,79 Gew.% CaO,
- 3,0 - 4,0 Gew.% Al₂O₃, vorzugsweise 3,46 Gew.% Al₂O₃,
- 3,0 - 4,0 Gew.% B₂O₃, vorzugsweise 3,18 Gew.% B₂O₃,
- 2,0 - 2,5 Gew.% ZnO, vorzugsweise 2,07 Gew.% ZnO,
- höchstens 0,1 Gew.% Fe₂O₃, vorzugsweise 0,01 Gew.% Fe₂O₃,
- höchstens 0,1 Gew.% TiO₂, vorzugsweise 0,01 Gew.% TiO₂,
- höchstens 0,06 Gew.% MgO, vorzugsweise 0,050 Gew.% MgO,
- höchstens 0,03 Gew.% BaO, vorzugsweise 0,025 Gew.% BaO,
- höchstens 0,02 Gew.% PbO, vorzugsweise 0,005 Gew.% PbO,
- höchstens 0,02 Gew.% Sb₂O₃,
- höchstens 0,01 Gew.% SrO, und
- höchstens 0,006 Gew.% Li₂O.

4. Kalibriermittel nach einem der vorhergehenden Ansprüche, **dadurch gekennzeichnet**, daß die Kalibriermittel in Form einer Kapillare ausgebildet sind, wobei der Kapillarendurchmesser im Bereich von 1,5 - 2,0 mm liegt.

5. Photometer, insbesondere zur Bilirubinbestimmung, **dadurch gekennzeichnet,** daß zur Kalibrierung Kalibriermittel aus Glas vorgesehen sind.

6. Photometer nach Anspruch 5, **dadurch gekennzeichnet, daß** die Kalibriermittel aus einem Glas gebildet sind, das folgende Zusammensetzung aufweist:
- 66,0 - 68,0 Gew.% SiO₂, vorzugsweise 66,91 Gew.% SiO₂,
- 10,0 - 11,0 Gew.% Na₂O, vorzugsweise 10,88 Gew.% Na₂O,
- 6,0 - 7,0 Gew.% CaO, vorzugsweise 6,79 Gew.% CaO,
- 3,0 - 4,0 Gew.% Al₂O₃, vorzugsweise 3,46 Gew.% Al₂O₃,
- 3,0 - 4,0 Gew.% B₂O₃, vorzugsweise 3,18 Gew.% B₂O₃,
- 2,0 - 2,5 Gew.% ZnO, vorzugsweise 2,07 Gew.% ZnO,
- höchstens 0,1 Gew.% Fe₂O₃, vorzugsweise 0,080 Gew.% Fe₂O₃,
- höchstens 0,1 Gew.% TiO₂, vorzugsweise 0,083 Gew.% TiO₂,
- höchstens 0,06 Gew.% MgO, vorzugsweise 0,050 Gew.% MgO,
- höchstens 0,03 Gew.% BaO, vorzugsweise 0,025 Gew.% BaO,
- höchstens 0,02 Gew.% PbO, vorzugsweise 0,017 Gew.% PbO,
- höchstens 0,02 Gew.% Sb₂O₃,
- höchstens 0,01 Gew.% SrO, und
- höchstens 0,006 Gew.% Li₂O.

7. Photometer nach Anspruch 5, **dadurch gekennzeichnet, daß** die Kalibriermittel aus einem Glas gebildet sind, das folgende Zusammensetzung aufweist:
- 66,0 - 68,0 Gew.% SiO₂, vorzugsweise 66,91 Gew.% SiO₂,
- 10,0 - 11,0 Gew.% Na₂O, vorzugsweise 10,88 Gew.% Na₂O,
- 6,0 - 7,0 Gew.% CaO, vorzugsweise 6,79 Gew.% CaO,
- 3,0 - 4,0 Gew.% Al₂O₃, vorzugsweise 3,46 Gew.% Al₂O₃,
- 3,0 - 4,0 Gew.% B₂O₃, vorzugsweise 3,18 Gew.% B₂O₃,
- 2,0 - 2,5 Gew.% ZnO, vorzugsweise 2,07 Gew.% ZnO,
- höchstens 0,1 Gew.% Fe₂O₃, vorzugsweise 0,01 Gew.% Fe₂O₃,
- höchstens 0,1 Gew.% TiO₂, vorzugsweise 0,01 Gew.% TiO₂,
- höchstens 0,06 Gew.% MgO, vorzugsweise 0,050 Gew.% MgO,
- höchstens 0,03 Gew.% BaO, vorzugsweise 0,025 Gew.% BaO,
- höchstens 0,02 Gew.% PbO, vorzugsweise 0,005 Gew.% PbO,
- höchstens 0,02 Gew.% Sb₂O₃,
- höchstens 0,01 Gew.% SrO, und
- höchstens 0,006 Gew.% Li₂O.

8. Photometer nach einem der Ansprüche 5 bis 7, **dadurch gekennzeichnet, daß** die Kalibriermittel in Form einer Kapillare ausgebildet sind, wobei der Kapillarendurchmesser im Bereich von 1,5 - 2,0 mm liegt.

## Claims

1. Calibration means for the photometric determination of bilirubin, characterised in that the calibration means are formed from glass.

2. Calibration means according to Claim 1, characterised in that the calibration means are formed from a glass having the following composition:
- 66.0 - 68.0% by wt. of SiO₂, preferably 66.91% by wt. of SiO₂,
- 10.0 - 11.0% by wt. of Na₂O, preferably 10.88% by wt. of Na₂O,
- 6.0 - 7.0% by wt. of CaO, preferably 6.79% by wt. of CaO,
- 3.0 - 4.0% by wt. of Al₂O₃, preferably 3.46% by wt. of Al₂O₃,
- 3.0 - 4.0% by wt. of B₂O₃, preferably 3.18% by wt. of B₂O₃,
- 2.0 - 2.5% by wt. of ZnO, preferably 2.07% by wt. of ZnO,
- at maximum 0.1% by wt. of Fe₂O₃, preferably 0.080% by wt. of Fe₂O₃,
- at maximum 0.1% by wt. of TiO₂, preferably 0.083% by wt. of TiO₂,
- at maximum 0.06% by wt. of MgO, preferably 0.050% by wt. MgO,
- at maximum 0.03% by wt. of BaO, preferably 0.025% by wt. of BaO,
- at maximum 0.02% by wt. PbO, preferably 0.017% by wt. of PbO,
- at maximum 0.02% by wt. of Sb₂O₃,
- at maximum 0.01% by wt. of SrO, and
- at maximum 0.006% by wt. of Li₂O.

3. Calibration means according to Claim 1, characterised in that the calibration means are formed from a glass having the following composition:
- 66.0 - 68.0% by wt. of SiO₂, preferably 66.91% by wt. of SiO₂,
- 10.0 - 11.0% by wt. of Na₂O, preferably 10.88% by wt. of Na₂O,
- 6.0 - 7.0% by wt. of CaO, preferably 6.79% by wt. of CaO,
- 3.0 - 4.0% by wt. of Al₂O₃, preferably 3.46% by wt. of Al₂O₃,
- 3.0 - 4.0% by wt. of B₂O₃, preferably 3.18% by wt. of B₂O₃,
- 2.0 - 2.5% by wt. of ZnO, preferably 2.07% by wt. of ZnO,
- at maximum 0.1% by wt. of Fe₂O₃, preferably 0.01% by wt. of Fe₂O₃,
- at maximum 0.1% by wt. of TiO₂, preferably 0.01% by wt. of TiO₂,
- at maximum 0.06% by wt. of MgO, preferably 0.050% by wt. of MgO,
- at maximum 0.03% by wt. of BaO, preferably 0.025% by wt. of BaO,
- at maximum 0.02% by wt. PbO, preferably 0.005% by wt. of PbO,
- at maximum 0.02% by wt. of Sb₂O₃,
- at maximum 0.01% by wt. of SrO, and
- at maximum 0.006% by wt. of Li₂O.

4. Calibration means according to one of the preceding claims, characterised in that the calibration means are constructed in the form of a capillary, the capillary diameter lying in the range of 1.5 - 2.0 mm.

5. Photometer, in particular for the determination of bilirubin, characterised in that calibration means made of glass are provided for calibration.

6. Photometer according to Claim 5, characterised in that the calibration means are formed from a glass having the following composition:
- 66.0 - 68.0% by wt. of SiO₂, preferably 66.91% by wt. of SiO₂,
- 10.0 - 11.0% by wt. of Na₂O, preferably 10.88% by wt. of Na₂O,
- 6.0 - 7.0% by wt. of CaO, preferably 6.79% by wt. of CaO,
- 3.0 - 4.0% by wt. of Al₂O₃, preferably 3.46% by wt. of Al₂O₃,
- 3.0 - 4.0% by wt. of B₂O₃, preferably 3.18% by wt. of B₂O₃,
- 2.0 - 2.5% by wt. of ZnO, preferably 2.07% by wt. of ZnO,
- at maximum 0.1% by wt. of Fe₂O₃, preferably 0.080% by wt. of Fe₂O₃,
- at maximum 0.1% by wt. of TiO₂, preferably 0.083% by wt. of TiO₂,
- at maximum 0.06% by wt. of MgO, preferably 0.050% by wt. MgO,
- at maximum 0.03% by wt. of BaO, preferably 0.025% by wt. of BaO,
- at maximum 0.02% by wt. PbO, preferably 0.017% by wt. of PbO,
- at maximum 0.02% by wt. of Sb₂O₃,
- at maximum 0.01% by wt. of SrO, and
- at maximum 0.006% by wt. of Li₂O.

7. Photometer according to Claim 5, characterised in that the calibration means are formed from a glass having the following composition:
- 60.0 - 68.0% by wt. of SiO₂, preferably 66.91% by wt. of SiO₂,
- 10.0 - 11.0% by wt. of Na₂O, preferably 10.88% by wt. of Na₂O,
- 6.0 - 7.0% by wt. of CaO, preferably 6.79% by wt. of CaO,
- 3.0 - 4.0% by wt. of Al₂O₃, preferably 3.46% by wt. of Al₂O₃,
- 3.0 - 4.0% by wt. of B₂O₃, preferably 3.18% by wt. of B₂O₃,
- 2.0 - 2.5% by wt. of ZnO, preferably 2.07% by wt. of ZnO,
- at maximum 0.1% by wt. of Fe₂O₃, preferably 0.01% by wt. of Fe₂O₃,
- at maximum 0.1% by wt. of TiO₂, preferably 0.01% by wt. of TiO₂,
- at maximum 0.06% by wt. of MgO, preferably 0.050% by wt. of MgO,
- at maximum 0.03% by wt. of BaO, preferably 0.025% by wt. of BaO,
- at maximum 0.02% by wt. PbO, preferably 0.005% by wt. of PbO,
- at maximum 0.02% by wt. of Sb₂O₃,
- at maximum 0.01% by wt. of SrO, and
- at maximum 0.006% by wt. of Li₂O.

8. Photometer according to one of Claims 5 to 7, characterised in that the calibration means are constructed in the form of a capillary, the capillary diameter lying in the range of 1.5 - 2.0 mm.

## Revendications

1. Agent de calibrage pour la détermination photométrique de la bilirubine, caractérisé en ce que les agents de calibrage sont formés de verre.

2. Agent de calibrage selon la revendication 1, caractérisé en ce que les agents de calibrage sont formés d'un verre qui présente la composition suivante:
- 66,0 - 68,0 % en poids de SiO₂, de préférence 66,91 % en poids de SiO₂,
- 10,0 - 11,0 % en poids de Na₂O, de préférence 10,88 % en poids de Na₂O,
- 6,0 - 7,0 % en poids de CaO, de préférence 6,79% en poids de CaO,
- 3,0 - 4,0 % en poids de Al₂O₃, de préférence 3,46 % en poids de Al₂O₃,
- 3,0 - 4,0 % en poids de B₂O₃, de préférence 3,18% en poids de B₂O₃,
- 2,0 - 2,5 % en poids de ZnO, de préférence 2,07% en poids de ZnO,
- au plus 0,1 % en poids de Fe₂O₃, de préférence 0,080 % en poids de Fe₂O₃,
- au plus 0,1 % en poids de TiO₂, de préférence 0,083 % en poids de TiO₂,
- au plus 0,06 % en poids de MgO, de préférence 0,050 % en poids de MgO,
- au plus 0,03 % en poids de BaO, de préférence 0,025 % en poids de BaO,
- au plus 0,02 % en poids de PbO, de préférence 0,017 % en poids de PbO,
- au plus 0,02 % en poids de Sb₂O₃,
- au plus 0,01 % en poids de SrO, et
- au plus 0,006 % en poids de Li₂O.

3. Agent de calibrage selon la revendication 1, caractérisé en ce que les agents de calibrage sont formés d'un verre qui présente la composition suivante:
- 66,0 - 68,0 % en poids de SiO₂, de préférence 66,91 % en poids de SiO₂,
- 10,0 - 11,0 % en poids de Na₂O, de préférence 10,88 % en poids de Na₂O,
- 6,0 - 7,0 % en poids de CaO, de préférence 6,79% en poids de CaO,
- 3,0 - 4,0 % en poids de Al₂O₃, de préférence 3,46 % en poids de Al₂O₃,
- 3,0 - 4,0 % en poids de B₂O₃, de préférence 3,18% en poids de B₂O₃,
- 2,0 - 2,5 % en poids de ZnO, de préférence 2,07% en poids de ZnO,
- au plus 0,1 % en poids de Fe₂O₃, de préférence 0,01 % en poids de Fe₂O₃,
- au plus 0,1 % en poids de TiO₂, de préférence 0,01 % en poids de TiO₂,
- au plus 0,06 % en poids de MgO, de préférence 0,050 % en poids de MgO,
- au plus 0,03 % en poids de BaO, de préférence 0,025 % en poids de BaO,
- au plus 0,02 % en poids de PbO, de préférence 0,005 % en poids de PbO,
- au plus 0,02 % en poids de Sb₂O₃,
- au plus 0,01 % en poids de SrO, et
- au plus 0,006 % en poids de Li₂O.

4. Agent de calibrage selon l'une quelconque des revendications précédentes, caractérisé en ce que les agents de calibrage sont conformés en capillaire, le diamètre du capillaire étant compris entre 1,5 et 2,0mm.

5. Photomètre, en particulier pour la détermination de la bilirubine, caractérisé en ce que, pour le calibrage, des agents de calibrage en verre sont prévus.

6. Photomètre selon la revendication 5, caractérisé en ce que les agents de calibrage sont formés d'un verre qui présente la composition suivante:
- 66,0 - 68,0 % en poids de SiO₂, de préférence 66,91 % en poids de SiO₂,
- 10,0 - 11,0 % en poids de Na₂O, de préférence 10,88 % en poids de Na₂O,
- 6,0 - 7,0 % en poids de CaO, de préférence 6,79% en poids de CaO,
- 3,0 - 4,0 % en poids de Al₂O₃, de préférence 3,46 % en poids de Al₂O₃,
- 3,0 - 4,0 % en poids de B₂O₃, de préférence 3,18% en poids de B₂O₃,
- 2,0 - 2,5 % en poids de ZnO, de préférence 2,07% en poids de ZnO,
- au plus 0,1 % en poids de Fe₂O₃, de préférence 0,080 % en poids de Fe₂O₃,
- au plus 0,1 % en poids de TiO₂, de préférence 0,083 % en poids de TiO₂,
- au plus 0,06 % en poids de MgO, de préférence 0,050 % en poids de MgO,
- au plus 0,03 % en poids de BaO, de préférence 0,025 % en poids de BaO,
- au plus 0,02 % en poids de PbO, de préférence 0,017 % en poids de PbO,
- au plus 0,02 % en poids de Sb₂O₃,
- au plus 0,01 % en poids de SrO, et
- au plus 0,006 % en poids de Li₂O.

7. Photomètre selon la revendication 5, caractérisé en ce que les agents de calibrage sont formés d'un verre qui présente la composition suivante:
- 66,0 - 68,0 % en poids de SiO₂, de préférence 66,91 % en poids de SiO₂,
- 10,0 - 11,0 % en poids de Na₂O, de préférence 10,88 % en poids de Na₂O,
- 6,0 - 7,0 % en poids de CaO, de préférence 6,79% en poids de CaO,
- 3,0 - 4,0 % en poids de Al₂O₃, de préférence 3,46 % en poids de Al₂O₃,
- 3,0 - 4,0 % en poids de B₂O₃, de préférence 3,18% en poids de B₂O₃,
- 2,0 - 2,5 % en poids de ZnO, de préférence 2,07% en poids de ZnO,
- au plus 0,1 % en poids de Fe₂O₃, de préférence 0,01 % en poids de Fe₂O₃,
- au plus 0,1 % en poids de TiO₂, de préférence 0,01 % en poids de TiO₂,
- au plus 0,06 % en poids de MgO, de préférence 0,050 % en poids de MgO,
- au plus 0,03 % en poids de BaO, de préférence 0,025 % en poids de BaO,
- au plus 0,02 % en poids de PbO, de préférence 0,005 % en poids de PbO,
- au plus 0,02 % en poids de Sb₂O₃,
- au plus 0,01 % en poids de SrO, et
- au plus 0,006 % en poids de Li₂O.

8. Photomètre selon l'une des revendications 5 à 7, caractérisé en ce que les agents de calibrage sont conformés en capillaire, le diamètre du capillaire étant compris entre 1,5 et 2,0 mm.
